**Europäisches Patentamt**

**European Patent Office**

**Office européen des brevets**

(11) Veröffentlichungsnummer: **0 040 430**
**B1**

(12) **EUROPÄISCHE PATENTSCHRIFT**

(45) Veröffentlichungstag der Patentschrift:
02.11.83

(51) Int. Cl.³: **C 12 N 9/04**, C 12 N 1/20 //
C12R1/085, C12Q1/54

(21) Anmeldenummer: 81103848.8

(22) Anmeldetag: 19.05.81

(54) Verfahren zur Gewinnung von Glucosedehydrogenase und hierfür geeignete Mikroorganismen.

(30) Priorität: 21.05.80 DE 3019450

(43) Veröffentlichungstag der Anmeldung:
25.11.81 Patentblatt 81/47

(45) Bekanntmachung des Hinweises auf die Patenterteilung:
02.11.83 Patentblatt 83/44

(84) Benannte Vertragsstaaten:
AT BE CH DE FR GB IT LI LU NL SE

(56) Entgegenhaltungen:
AGRIC. BIOL. CHEM., Band 43, Nr. 2, 1979 A. YOKOTA et al.: "Reactivation of Inactivated D-Glucose Dehydrogenase of a Bacillus Species by Pyridine and Adenine Nucleotides", Seiten 271-278
CHEMICAL ABSTRACTS, Band 83, Nr. 23, 8. Dezember 1975, Zusammenfassung Nr. 190059k, Seite 196 COLUMBUS OHIO (US)
CHEMICAL ABSTRACTS, Band 57, Nr. 2, 23. Juli 1962, Spalte 2654f COLUMBUS OHIO (US)

(73) Patentinhaber: BOEHRINGER MANNHEIM GMBH,
Patentabteilung, Abt. E Sandhofer
Strasse 112-132 Postfach 31 01 20,
D-6800 Mannheim 31 Waldhof (DE)

(72) Erfinder: von Hoerschelmann, Detlef, Dr.,
Fasanenweg 7, D-8121 Wielenbach (DE)
Erfinder: Seidel, Hans, Dr., Waxensteinstrasse 6,
D-8132 Tutzing (DE)
Erfinder: Berger, Gerhard, Blombergstrasse 17,
D-8127 Iffeldorf (DE)
Erfinder: Masuth, Armin, Hörnerweg 46,
D-2000 Hamburg 26 (DE)
Erfinder: Beaucamp, Klaus, Dr.,
Von-Kühlmann-Strasse 15, D-8132 Tutzing (DE)
Erfinder: Gruber, Wolfgang, Dr., Am Oberanger 7,
D-8132 Tutzing-Unterzeismering (DE)

(74) Vertreter: Weickmann, Heinrich, Dipl.-Ing. et al,
Patentanwälte Dipl.-Ing. H. Weickmann Dipl.-Phys.Dr. K.
Fincke Dipl.-Ing. F.A. Weickmann Dipl.-Chem. B. Huber
Dr.-Ing. H. Liska Möhlstrasse 22,
D-8000 München 86 (DE)

Verfahren zur Gewinnung von Glucosedehydrogenase und hierfür geeignete Mikroorganismen

Die Erfindung betrifft ein Verfahren zur Gewinnung von Glucosedehydrogenase und einen hierfür besonders geeigneten neuen Mikroorganismus.

Glucosedehydrogenase, E.C. 1.1.1.47, katalysiert folgende Reaktion:

$$\beta\text{-D-Glucose} + NAD \xrightarrow{\text{Glucosedehydrogenase}} \text{D-Gluconolacton} + NADH_2$$

Da sich $NADH_2$ leicht bestimmen lässt, eignet sich diese Umsetzung zur analytischen Bestimmung von Glucose.

Ein Nachteil dieses Verfahrens liegt darin, dass das Enzym bisher nur schlecht zugänglich und daher teuer war. Dies beruht einesteils auf schwer entfernbaren störenden Verunreinigungen (resistente Sporen, NADH-Oxidasen), andererseits auf zu geringen Ausbeuten. Zwar ist es aus „Abstracts", 5th International Fermentation Symposium, Berlin 1976, S. 260 bekannt, durch Züchten von Bacillus megaterium M 1286 die Sporenbildung zu unterdrücken. Die Ausbeute soll dabei ca. 2 U/ml betragen. Eigene Versuche ergaben jedoch nur höchstens 0,25 U/ml und sehr starke Verunreinigung mit NADH-Oxidasen.

Bekannt war es auch, einen Bacillus cereus-Stamm zu verwenden, der jedoch nur etwa 0,1 U Glucosedehydrogenase/ml Kultur erzeugte und starke Sporenbildung aufwies.

Der Erfindung liegt die Aufgabe zugrunde, das bekannte Verfahren zur Herstellung von NAD⁺-abhängiger Glucosedehydrogenase zu verbessern, insbesondere hinsichtlich der Enzymausbeute, des weiteren hinsichtlich der Sporulation (Sporenbildung bis zur Bildung maximaler Glucosedehydrogenase-Aktivität ist unerwünscht) sowie Abtrennbarkeit störender Fremdaktivitäten, insbesondere von NADH-Oxidasen.

Gelöst wird diese Aufgabe erfindungsgemäss durch ein Verfahren zur mikrobiologischen Herstellung von NAD⁺-abhängiger Glucosedehydrogenase, welches dadurch gekennzeichnet ist, dass man Bacillus cereus DSM 1644 oder eine NAD⁺-abhängige Glucosedehydrogenase bildende, aus Bacillus cereus DSM 1730, Bacillus cereus DSM 345 oder Bacillus cereus ATCC 10987 künstlich hergesellte Bacillus cereus lad-Mutante züchtet, deren Sporenentwicklung bis zur Entwicklungsstufe III normal verläuft, dann aber bis ca. zur 20. Stunde gehemmt ist, um danach bis zur Entwicklung hitzeresistenter Sporen fortzuschreiten.

NAD⁺-abhängige Glucosedehydrogenase bildende, aus DSM 1730, DSM 345 oder ATCC 10987 erhältliche Bacillus cereus lad-Mutanten, deren Sporenentwicklung bis zur Entwicklungsstufe III normal verläuft, dann aber bis ca. zur 20. Stunde gehemmt ist, um danach bis zur Entwicklung hitzeresistenter Sporen fortzuschreiten, sind neu und können durch ein Mutierungsverfahren aus dem Ausgangsstamm erhalten werden.

Bevorzugt wird als Ausgangsstamm Bacillus cereus DSM 1730.

Die Herstellung der genannten Bacillus cereus lad-Mutanten kann folgendermassen durchgeführt werden:

Der Ausgangsstamm wird zur Mutation in Gegenwart von Methansulfonsäuremethyl- oder -äthylester 10 bis 90 min bei 28° C gebrütet. Dann wird die Mutagenese durch Waschen in Phosphatpuffersaline gestoppt und das Mutagenisat in Oberflächenkultur aufgetrennt. Dabei wurde nach der Mutagenese eine Übernachtkultur hergestellt, die nach 12stündiger Inkubation mikroskopiert wurde und eine möglichst asynchrone Phase, in der simultan sporenfreie, endosporenhaltige Zellen sowie die ersten freien Sporen sichtbar waren, abgetrennt und für einen frischen Ansatz verwendet. Nach 5 bis 7 Passagen erhielt man so, ausgehend von Bacillus cereus DSM 1730 den reinen Stamm Bacillus cereus DSM 1644, welcher ebenfalls Gegenstand der Erfindung ist.

Die taxonomische Bestimmung von DSM 1644 ergab, dass der Organismus aus Einzelzellen mit Neigung zur Kettenbildung besteht. Zellgrösse 1×4 bis 6 μ (wuchsphasenabhängig). Die Sporen sind oval in der Form und polar angeordnet. Grampositiv. Wachstum bei 25 bis 40° C aerob und anaerob. β-Hämolyse auf Blutagar. Weitere positive Reaktionen: Glucose, Maltose, Salicin, Nitrat, Voges-Proskauer, Citrat, Katalase.

Negative Tests: Indol, Oxidase, Lysindecarboxylase, Lactose, Arabinose, Saccharose, Mannose und Xylase.

Die Definition der Wachstumsphase entspricht „Biochem. J.", 109, 819 (1968) bzw. „Adv. Genet", 18, 69 (1976).

Für die Züchtung eignen sich die bekannten Nährböden, welche für die Gattung Bacillus geeignet sind. Bevorzugt wird ein Nährmedium, welches als Kohlenstoffquelle anstelle von Glucose Glycerin enthält. Ein besonders bevorzugtes Nährmedium enthält pro Liter 6 bis 50 g Glycerin, 1 bis 10 g Hefeextrakt, 1 bis 50 g Pepton, 1 bis 20 g Ammoniumsulfat, 0,2 bis 5 g Dikaliumphosphat, 0,1 bis 5 g Magnesiumsulfat, 1 bis 10 g NaCl sowie Spurenelemente (Mn, Zn, Cu, Ca, Fe). Der pH-Wert wird zwischen 7 und 8 eingestellt, zweckmässig durch Zugabe von Natronlauge.

Zur Erzielung einer maximalen Glucosedehydrogenase-Aktivität wird 12 bis 20 h (bezogen auf 30° C) aerob gezüchtet. Dann wird die Biomasse abgetrennt und das Enzym gewonnen.

Die Glucosedehydrogenase findet sich normalerweise intrazellulär und die abgetrennten Zellen werden daher nach üblichen Methoden zur Solubilisierung des Enzyms aufgeschlossen. Geeignete Methoden sind Ultraschallaufschluss, Lysozymaufschluss, Vibrogenmühle und andere. Die Enzymausbeute beträgt bis zu 2000 U/l Kulturmedium. Die weitere Aufreinigung kann nach bekannten Methoden erfolgen, z.B. wie in „J. of Bacteriology", 132, 282-293 (1977) beschrieben.

Ein besonderer Vorzug des erfindungsgemässen Verfahrens besteht darin, dass infolge der verzögerten Sporulation, die während der Züchtung bis zur Erzielung einer maximalen Enzymausbeute nicht zur Bildung hitzeresistenter Sporen führt, Kontaminationsprobleme vermieden werden. Dies vereinfacht die Züchtung und Gewinnung des Enzyms wesentlich.

Beim Ausgangsstamm *Bacillus cereus* DSM 1730 tritt demgegenüber bereits nach 15 h eine so massive Sporulation auf, dass die daraus gewonnene Biomasse stets eine erhebliche Menge an hitzeresistenten Sporen enthält.

Dies zeigt, dass erfingungsgemäss die Enzymausbeute gesteigert werden kann und gleichzeitig das Verfahren wesentlich vereinfacht wird. Als zusätzlicher Vorteil kommt hinzu, dass sich unerwünschte Enzymnebenaktivitäten, insbesondere die bei der Verwendung zur Glucosebestimmung besonders störende NADH-Oxidaseaktivität sehr leicht abtrennen lassen, beispielsweise durch einen Erhitzungsschritt.

Das folgende Beispiel erläutert die Erfindung.

*Beispiel*

In ein Nährmedium folgender Zusammensetzung 2,8 g Hefeextrakt, 4 g Ammoniumsulfat, 1,0 g $K_2HPO_4$, 0,8 g $MgSO_4$, 4,8 g NaCl, 5 g Glycerin (87%-ig), 1,6 g Pepton sowie Spurenmengen $MnSO_4$, $ZnSO_4$, $CuSO_4$, $CaCl_2$ und $FeCl_3$, pH 7,4, wurde nach Sterilisation mit *Bacillus cereus* DSM 1644 beimpft. Nach 8stündiger Bebrütung bei 30° C wurde mit der so erhaltenen Vorkultur das in gleicher Weise zusammengesetzte Medium der Hauptkultur mit 1% Vorkultur belüftet. In der Hauptkultur wurde 19 h bei 30° C unter Belüftung in der Schüttelkultur gezüchtet. Die Glucosedehydrogenase-Bestimmung ergibt zu diesem Zeitpunkt 1800 U/l. Die Zellmasse wird dann abzentrifugiert und eingefroren. Nach Auftauen wird mit Ultraschall aufgeschlossen und vom Unlöslichen abgetrennt.

Die Aktivitätsbestimmung wird wie folgt durchgeführt:

*Testansatz:*

2,1 ml Trismanganpuffer, 0,05 M + 12 mg/l $MnSO_4 \cdot H_2O$, pH 8,5,

0,3 ml Glucoselösung 1 M,

0,4 ml NAD-Lösung c = 10 mg/ml.

Die Reaktion wird durch Probezusatz (Rohextrakt) gestartet. Temperatur: 25° C; gemessen wird die Extinktionszunahme bei 365 nm.

**Patentansprüche**

1. Verfahren zur mikrobiologischen Herstellung von NAD⁺-abhängiger Glucosedehydrogenase, dadurch gekennzeichnet, dass man *Bacillus cereus* DSM 1644 oder eine NAD⁺-abhängige Glucosedehydrogenase bildende, aus *Bacillus cereus* DSM 1730, *Bacillus cereus* DSM 345 oder *Bacillus cereus* ATCC 10987 künstlich erhaltene *Bacillus cereus* lad-Mutante züchtet, deren Sporenentwicklung bis zur Entwicklungsstufe III normal verläuft, dann aber bis ca. zur 20. Stunde gehemmt ist, um danach bis zur Entwicklung hitzeresistenter Sporen fortzuschreiten.

2. Verfahren nach Anspruch 1, dadurch gekennzeichnet, dass man in einem Nährmedium züchtet, dessen Kohlenstoffquelle überwiegend oder vollständig aus Glycerin besteht.

3. Verfahren nach Anspruch 2, dadurch gekennzeichnet, dass man in einem Nährmedium züchtet, welches pro Liter 5 bis 50 g Glycerin, 1 bis 10 g Hefeextrakt, 1 bis 50 g Pepton, 1 bis 20 g Ammoniumsulfat, 0,2 bis 5 g Dikaliumphosphat, 0,1 bis 5 g Magnesiumsulfat, 1 bis 10 g NaCl sowie Spurenelemente enthält.

4. NAD⁺-abhängige Glucosedehydrogenase bildende, aus DSM 1730, DSM 345 oder ATCC 10987 erhältliche *Bacillus cereus* lad-Mutanten, deren Sporenentwicklung bis zur Entwicklungsstufe III normal verläuft, dann aber bis ca. zur 20. Stunde gehemmt ist, um danach bis zur Entwicklung hitzeresistenter Sporen fortzuschreiten.

5. *Bacillus cereus* DSM 1644.

**Claims**

1. Process for the microbiological production of NAD⁺-dependent glucose dehydrogenase, characterised in that one cultures *Bacillus cereus* DSM 1644 or an NAD⁺-dependent, glucose dehydrogenase-forming *Bacillus cereus* lad-mutant obtained artificially from *Bacillus cereus* DSM 1730, *Bacillus cereus* DSM 345 or *Bacillus cereus* ATCC 10987, the spore development of which proceeds normally up to the development stage III but is then inhibited up to about the 20th hour and thereafter continues up to the development of heat-resistant spores.

2. Process according to claim 1, characterised in that one cultures in a nutrient medium, the carbon source of which consists preponderantly or completely of glycerol.

3. Process according to claim 2, characterised in that one cultures in a nutrient medium which, per litre, contains 5 to 50 g glycerol, 1 to 10 g yeast extract, 1 to 50 g peptone, 1 to 20 g ammonium sulphate, 0.2 to 5 g dipotassium phosphate, 0.1 to 5 g magnesium sulphate, 1 to 10 g NaCl, as well as trace elements.

4. NAD⁺-dependent, glucose dehydrogenase-forming *Bacillus cereus* lad-mutants obtainable from DSM 1730, DSM 345 or ATCC 10987, the spore development of which proceeds normally up to the development stage III but is then inhibited up to about the 20th hour and thereafter continues up to the development of heat-resistant spores.

5. *Bacillus cereus* DSM 1644.

**Revendications**

1. Procédé de préparation microbiologique de glucose-déshydrogénase dépendant de NAD⁺, caractérisé en ce qu'on cultive *Bacillus cereus* 1644, ou un lad-mutant de *Bacillus cereus* formant de la glucose-déshydrogénase dépendante de NAD+, obtenu artificiellement à partir de *Bacillus cereus* DSM 1730, *Bacillus cereus* DSM 345 ou *Bacillus cereus* ATCC 10987, dont le développement des spores évolue normalement jusqu'au stade de développement III, mais est ensuite inhibé jusqu'à la 20e heure environ, pour se poursuivre ensuite jusqu'au développement de spores résistantes à la chaleur.

2. Procédé selon la revendication 1, caractérisé en ce qu'on cultive dans un milieu nutritif dont la source de carbone se compose principalement ou totalement de glycérol.

3. Procédé suivant la revendication 2, caractérisé en ce qu'on cultive dans un milieu nutritif qui contient par litre 5 à 50 g de glycérol, 1 à 10 g d'extrait de levure, 1 à 50 g de peptone, 1 à 20 g de sulfate d'ammonium, 0,2 à 5 g de phosphate dipotassique, 0,1 à 5 g de sulfate de magnésium, 1 à 10 g de NaCl, ainsi que des oligo-éléments.

4. Lad-mutants de *Bacillus cereus* formant de la glucose-déshydrogénase dépendante de NAD+, pouvant être obtenus à partir de DSM 1730, DSM 345 ou ATCC 10987 dont le développement de spores évolue normalement jusqu'au stade de développement III, mais est ensuite inhibé jusqu'à la 20e heure environ, pour se poursuivre ensuite jusqu'au développement de spores résistant à la chaleur.

5. *Basillus cereus* DSM 1644.